# EUROPÄISCHE PATENTSCHRIFT

(11) **EP 1 045 863 B1**
(45) Veröffentlichungstag und Bekanntmachung des Hinweises auf die Patenterteilung: **02.04.2003**
(21) Anmeldenummer: 98961184.3
(22) Anmeldetag: 11.11.1998
(51) Int. Cl.: C08B 37/00, A61K 47/48

(54) **ENTGIFTUNG VON PHARMAZEUTISCHEN WIRKSTOFFEN DURCH CYCLODEXTRIN-OLIGOMERE**
DETOXICATION OF ACTIVE PHARMACEUTICAL SUBSTANCES USING CYCLODEXTRINE OLIGOMERS
DETOXICATION DE SUBSTANCES PHARMACEUTIQUES ACTIVES A L'AIDE D'OLIGOMERES DE CYCLODEXTRINES

(30) Priorität: 11.11.1997 DE 19749801; 19.05.1998 DE 19822416
(43) Veröffentlichungstag der Anmeldung: 25.10.2000
(73) Patentinhaber: Ceramoptec Inc., East Longmeadow, MA 01028 (US)
(72) Erfinder: MOSER, Jörg G., D-40591 Düsseldorf (DE)
(74) Vertreter: Meyers, Hans-Wilhelm, Dr.Dipl.-Chem.
(86) Internationale Anmeldenummer: EP9807229
(87) Internationale Veröffentlichungsnummer: WO99024474

(56) Entgegenhaltungen:
- WO-A-91/13100
- A. RUEBNER ET AL.: "Dimeric Cyclodextrin Carriers with High Binding Affinity to Porphyrinoid Photosensitizers" JOURNAL OF INCLUSION PHENOMENA AND MOLECULAR RECOGNITION IN CHEMISTRY, Bd. 27, 1997, Seiten 69-84, XP002097235 in der Anmeldung erwähnt
- J. G. MOSER ET AL.: "Cyclodextrin Dimers Used to Prevent Side Effects of Photochemotherapy and General Tumor Therapy" JOURNAL OF INCLUSION PHENOMENA AND MOLECULAR RECOGNITION IN CHEMISTRY, Bd. 25, 1996, Seiten 29-34, XP002097236
- T. CSERHATI UND J. HOLLO: "Interaction of Taxol and other Anticancer Drugs with Hydroxypropyl-beta-cyclodextrin" INTERNATIONAL JOURNAL OF PHARMACEUTICS, Bd. 108, 1994, Seiten 69-75, XP000770247

## Beschreibung

Die vorliegende Erfindung betrifft über einen Spacer verbrückte Cyclodextrin-Oligomere und Komplexe dieser Cyclodextrin-Oligomere mit pharmazeutischen Wirkstoffen.

Cyclodextrine sind ringförmige Glucosepolymere, die in Abhängigkeit von der Anzahl der Glucose-Einheiten (6 bis 8) als α-, β-bzw. γ-Cyclodextrine bezeichnet werden. Innerhalb des Oligoglucoserings befindet sich eine lipophile Kavität. Es ist bekannt, daß lipophile Substanzen in diese Kavität eingeschlossen werden können. Cyclodextrine werden unter anderem dazu genutzt, Verbindungen mit geringer Löslichkeit durch Komplexierung mit Cyclodextrin in einen löslichen Komplex zu überführen.

Breslow, R. et al. beschreiben in Tetrahedron Vol. 51 2 (1995), S. 377-388 Cyclodextrin-Dimere, die Substrate mit einer korrekten Geometrie in wäßrigen Lösungen binden können.

Ruebner, A. et al. beschreiben im Journal of Inclusion Phenomena and Molecular Recognition in Chemistry 27 (1997), S. 69-84 dimere Cyclodextrine mit hohen Bindungsaffinitäten für Porphyrinoid-Photosensitizer als Carrier-System für die Anwendung von Wirkstoffen in der photodynamischen Krebstherapie.

Fujita, K. et al. beschreiben in Tetrahedron Letters 25 (1984), S. 5533-5536 die Herstellung von Ditosylaten von β-Cyclodextrin und ihre Aufreinigung durch Umkehrphasen-Chromatographie.

Tabushi, I. et al. beschreiben in Tetrahedron Letters 18 (1977), S. 1527-1530 die spezifische Bifunktionalisierung von Cyclodextrin.

Feederle, R. et al. beschreiben in Arch. Microbiol. 165 (1996), S. 206-212 die Aufreinigung und Charakterisierung des Enzyms Cyclodextrinase aus *Klebsiella oxytoca*.

Manunza, B. et al. beschreiben in einer elektronischen Veröffentlichung, erreichbar unter *http:*//*antas.agraria.uniss.it*/*electronic_papers*/*eccc3*/*bcd*/*welcome.htm* eine molekulardynamische Studie der Struktur und internen Bewegung von solvatisiertem β-Cyclodextrin.

Sharma, U.S., et al. beschreiben im Journal of Pharmaceutical Sciences 84 (1995), S. 1223-1230 die pharmazeutischen und physikalischen Eigenschaften von Paclitaxel (Taxol)-Komplexen mit Cyclodextrinen.

Savitsky, A.P. et al. beschreiben in SPIE Biomedical Optics 3191 (1997), S. 343-353 ein Avidin-Biotin-System zum gezielten Transport von Photosensitizern und anderen cytotoxischen Agentien in Tumorgewebe.

Fazio, F. & Paganelli, G. beschreiben in Eur. J. Nucl. Med. 20 (1993), S. 1138-1140, daß derartige Biotin-Avidin-Systeme eine hochspezifische Tumormarkierung erlauben.

Die vorliegende Erfindung hat sich die Aufgabe gestellt, Cyclodextrin-Oligomere bereitzustellen, die sich aufgrund ihrer Geometrie zum Einschluß pharmazeutischer Wirkstoffe eignen.

Gelöst wird die Aufgabe durch Cyclodextrin-Oligomere mit den Merkmalen des Patentanspruchs 1.

Bei den erfindungsgemäßen Cyclodextrin-Oligomeren handelt es sich um zwei durch einen Spacer an der Sekundärseite verbundene Cyclodextrine, wobei der Spacer die Einheit B umfaßt, die ein starres und bevorzugt hydrophiles Strukturelement ist. In einer Ausführungsform weisen die Cyclodextrin-Oligomere die allgemeine Formel

CD-X-A-X-B-X-A-X-CD

auf, wobei
X jeweils unabhängig voneinander ausgewählt wird aus -NH-, -O-, -S-, -CO- oder kovalenter Bindung;
A jeweils ein aliphatischer C₂ bis C₄-Rest oder eine kovalente Bindung ist;
CD jeweils ein über die Sekundärseite gebundenes Cyclodextrin darstellt und
die Einheit B ausgewählt ist aus Melamin, Trimesinsäure, Alizarintetracarbonsäure oder Tetraamino-Alizarin bzw. Cyclodextrine wie α-Cyclodextrin, β-Cyclodextrin oder γ-Cyclodextrin, wobei die Cyclodextrine über die Primärseite gebunden sind.

Beispielhaft seien für die erfindungsgemäßen Cyclodextrin-Oligomere die Verbindungen
Di-β-CD(Terephthaldiamid),
Di-β-CD(-NHCO(Trimesyl)CONH-),
Di-β-CD(-NH-(C₂H₄)-NHCO(Trimesyl)CONH-(C₂H₄)-NH-),
Di-β-CD(-NH-(C₂H₄)-SH-[6-α-CD-6]-SH-(C₂H₄)-NH-),
Di-β-CD(-NH-(C₂H₄)-SH-[6-(β,γ)-CD-6]-SH-(C₂H₄)-NH-) oder
Di-β-CD(-NH-(C₃H₆)-NHCO(Trimesyl)CONH-(C₃H₆)-NH-) genannt.

Die erfindungsgemäßen Cyclodextrin-Oligomere sind erhältlich durch Tosylierung der OH-Gruppen auf der Sekundärseite von Cyclodextrinen und anschließende Umsetzung mit kurzen bifunktionellen Spacergruppen. Die so erhaltenen funktionalisierten Cyclodextrine können mit bifunktionellen Reagenzien zu Dimeren umgesetzt werden. Entsprechende Synthesevorschriften finden sich in Ruebner A. et al. (a.a.O.). Alternativ können hierzu auch Carbonsäurefunktionen in die Cyclodextrine eingeführt werden, indem die regiospezifischen Tosylate mit Amino- oder Mercaptocarbonsäuren wie 3-Mercaptopropionsäure, 4-Aminobutansäure umgesetzt werden.

Bindungskonstanten können in Kompetition mit 6-(*p*-Toluidino)-2-naphthalensulfonsäure (TNS) bestimmt werden. Die Messung erfolgt durch Fluoreszenzspektroskopie gemäß Ruebner et al. (a.a.O.).

Vorzugsweise trägt das Cyclodextrin-Oligomer zusätzlich mindestens eine, bevorzugt zwei Affinitätsgruppierungen, die mit molekularen Zielstrukturen in Wechselwirkung treten können.

Prinzipiell kann die mindestens eine Affinitätsgruppierung eine Bindungsfähigkeit für ein gewebespezifisches Antigen als molekulare Zielstruktur aufweisen. Im Sinne der von Fazio & Paganelli (a.a.O.) angestrebten polyphasischen Applikationsroute wird jedoch eine indirekte gewebespezifische Bindung bevorzugt: die Affinitätsgruppierung erkennt eine Zielstruktur, die zuvor gewebespezifisch am gewünschten Wirkort angebracht wurde.

Ein möglicher Wirkort ist beispielsweise ein Tumor. Ein gewebe- bzw. tumorspezifischer Antikörper, der die Zielstruktur für die Affinitätsgruppierung der erfindungsgemäßen Substanz trägt, kann somit - im Sinne einer mehrphasigen Tumortherapie - zunächst einmal oder mehrmals in den Organismus gebracht werden, um sich am Wirkort anzureichern. Anschließend kann durch Gabe eines Komplexes aus einem pharmazeutischen Wirkstoff und der erfindungsgemäßen Substanz mit der Affinitätsgruppierung eine zielgerichtete Anreicherung des pharmazeutischen Wirkstoffes am Wirkort erreicht werden. Der pharmazeutische Wirkstoff kann dann am Wirkort freigesetzt werden. Dies wird beispielsweise durch Zerstörung der Cyclodextrin-Einheit(en) am Wirkort erreicht. Als Antikörper können poly- oder monoklonale Antikörper, aber auch Antikörperfragmente wie Fab- oder F(ab)₂-Fragmente und künstliche Antikörper wie scFv-Fragmente verwendet werden. Als geeignete Affinitätsgruppierungen und Zielstrukturen kommen eine Vielzahl von Partnern, beispielsweise Biotin/Avidin, Biotin/Streptavidin oder Enzym/Inhibitorsysteme in Frage. Bevorzugte Affinitätsgruppierungen sind Biotinylreste und Digoxin-/Digoxigeninreste.

Gegenstand der Erfindung ist daher weiterhin der Komplex aus einem pharmazeutischen Wirkstoff und den erfindungsgemäßen Cyclodextrin-Oligomeren. Diese physikalischen, äußerlich stark hydrophilen Einschluß-Komplexe dienen der Verhinderung der Aufnahme des komplexierten pharmazeutischen Wirkstoffes in Körperzellen mit dem Ziel, die Nebenwirkungen beispielsweise von Tumortherapeutika zu vermindern oder auszuschließen. Der pharmazeutische Wirkstoff hat bevorzugt ein hohes Nebenwirkungspotential und ist daher in freier Form nur limitiert anwendbar. Geeignete pharmazeutische Wirkstoffe sind Mitosehemmer, Tumortherapeutika und Photochemotherapeutika, insbesondere Taxol, ein Taxolderivat, Cholchizin, Colchemid, 3¹,8¹-(di-t-butyl-phenoxy)porphyrine, Chlortrianisen, Tamoxifen, Vinblastin, Vincristin, Docetaxel.

Soweit die Cyclodextrin-Oligomere eine weitere Affinitätsgruppierung tragen, können sie zur zielgerichteten polyphasischen Applikation eines pharmazeutischen Wirkstoffes dienen. Überraschenderweise können durch dieses Verfahren die Nebenwirkungen einer Therapie verringert werden. Zusätzlich ist eine erhebliche Dosisreduktion bis zu einem Faktor von 10000 möglich.

Auch Arzneimittel, die mindestens ein erfindungsgemäßes Cyclodextrin-Oligomer oder mindestens einen erfindungsgemäßen Komplex enthalten, sind Gegenstand dieser Erfindung. Diese Arzneimittel eignen sich insbesondere zur Behandlung von Tumorerkrankungen wie Blasentumore, Mammacarcinome, Uteruscarcinome, Oesophaguscarcinome, Magencarcinome, Kopf-Hals-Carcinome, Nasopharynxcarcinom, Hepatocell. Carcinome, Lungencarcinome, Lymphome, Melanome, Ovarialcarcinome und Prostatacarcinome.

Indikation, Dosierung und Behandlungserfolge bei Tumorerkrankungen sind beschrieben in: Proceedings ASCO, Vol. 16 (1997) (Denver CO, USA).

Die Verknüpfung der Cyclodextrine auf der Sekundärseite führt dazu, daß die Kavitäten der Cyclodextrine zueinander gerichtet sind. Bevorzugt handelt es sich um β-Cyclodextrine. Der Abstand zwischen den Cyclodextrin-Einheiten wird durch die Auswahl des Spacers bestimmt. In Abhängigkeit von der Größe der einzuschließenden Verbindung wird der Spacer so gewählt, daß der Abstand zwischen den mindestens zwei Kavitäten in etwa dem Abstand zweier hydrophober Gruppen des pharmazeutischen Wirkstoffes entspricht. Bevorzugte Spacerabstände liegen im Bereich von etwa 0,6 bis 2 nm mit den bevorzugten Abständen im Bereich von etwa 0,8, 1,0, 1,2, 1,4, 1,6 und 1,8 nm. Entsprechende Verbindungen sind in der folgenden Tabelle gezeigt.

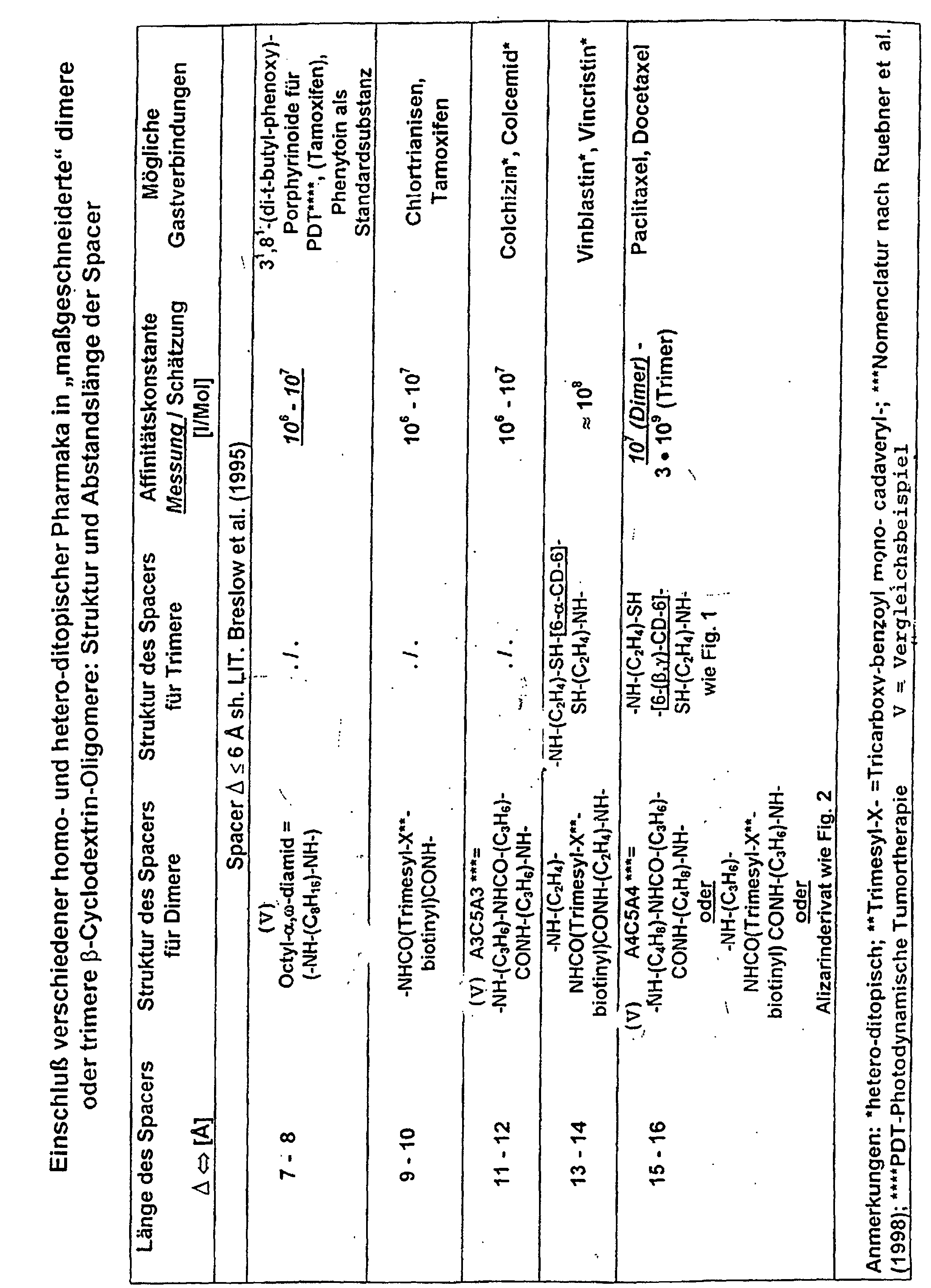

Der Spacer zwischen den beiden Cyclodextrin-Einheiten ist starr, d.h. der Spacer hat nur eine geringe Zahl an Bindungen, die frei um die Bindungsachse rotieren können. Vorzugsweise enthält der Spacer eine weitere Cyclodextrin-Einheit, die bevorzugt über die Primärseite mit kurzen aliphatischen Spacern an die kömplexierenden Cyclodextrine gebunden sind.

Entsprechende starre Spacer können auch durch den Einsatz entsprechend funktionalisierter (Hetero-)Aromaten erreicht werden. Starre Spacer verhindern zum Beispiel eine zu hohe konformative Beweglichkeit, wie ein Verdrillen der beiden an der Bindung beteiligten Cyclodextrine. Dies führt zu vergleichsweise hohen Bindungskonstanten, wie sie in der vorstehenden Tabelle angegeben sind. Durch den Einsatz starrer Spacer werden Affinitätskonstanten im Bereich von größer 10⁶, bevorzugt größer 10⁷ und noch mehr bevorzugt größer 10⁸ l/mol erreicht. Starre Spacer sind also solche, die zusammen mit an der Bindung beteiligten Cyclodextrinen und geeigneten Gastverbindungen entsprechend hohe Stabilitätskonstanten der Komplexe erreichen.

Die erfindungsgemäßen Komplexe können beispielsweise durch Einwirken von Cyclodextrinase aus Klebsiella oxytoca zerstört werden, so daß der komplexierte pharmazeutische Wirkstoff freigesetzt wird. Alternativ können in den Spacern funktionale Gruppen enthalten sein, die ein Aufbrechen der Bindung am Zielort erleichtern.

Figur 1 zeigt ein Schema für die Synthese eines trimeren Cyclodextrins.

Figur 2 zeigt die Struktur von Alizarin-überbrückten Cyclodextrinen, die mit zwei Biotinyl-Affinitätsgruppen derivatisiert sind.

Figur 3 zeigt das Fluoreszenzspektrum des Ansatzes gemäß beispiel 6. Der verkapselte Paclitaxel ist monomerisiert.

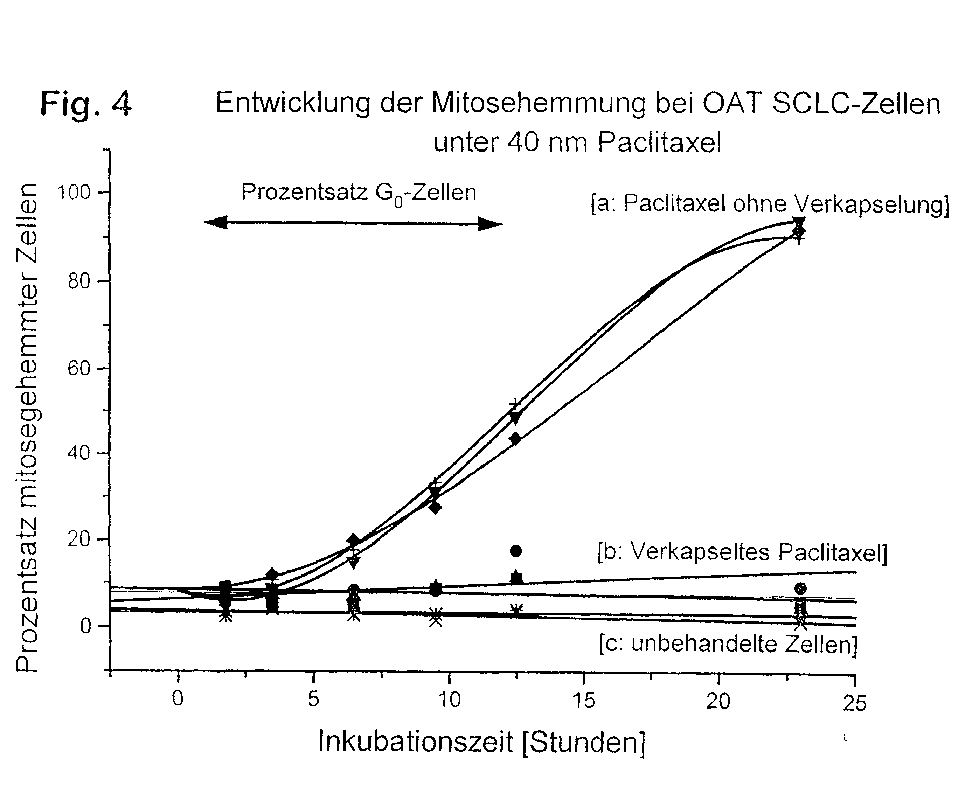

Figur 4 zeigt den Einfluß auf die Mitose von OAT-Zellen durch freies Taxol und komplexiertes Taxol gemäß Beispiel 8. Dabei bedeuten die oberen drei Linien (Kreuz, Dreieck, Raute) die Wirkung von unverkapseltem Taxol, die Kurven Quadrat, Dreieck und Kreis die Wirkung von verkapseltem Taxol und die drei Kurven (Kreuz) unbehandelte Zellen.

Figur 5 zeigt die Reaktivierung von Taxol aus dem Komplex durch Behandlung mit Cyclodextrinase gemäß Beispiel 8.

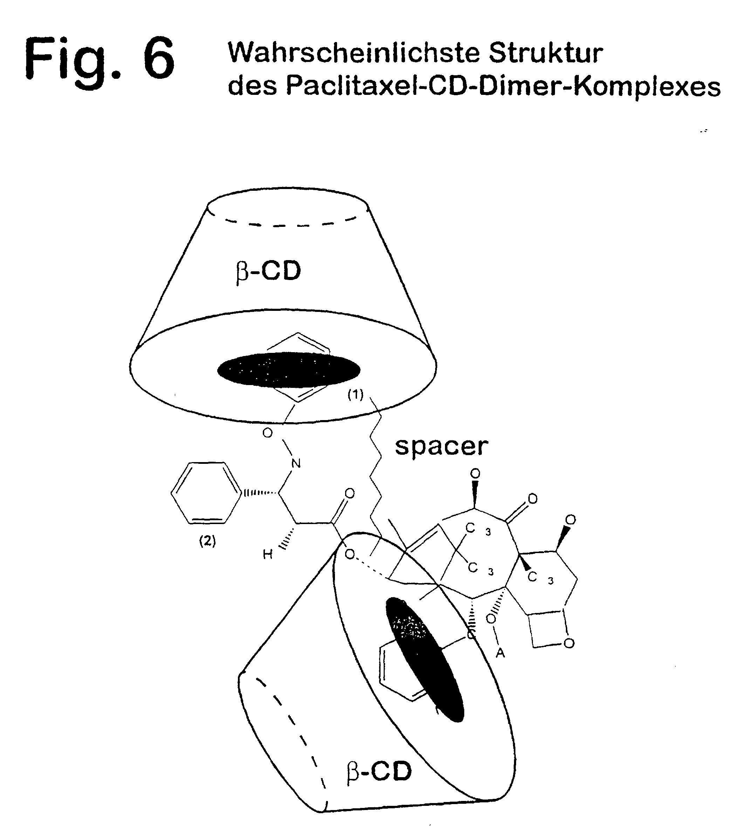

Figur 6 zeigt die wahrscheinlichste Struktur für den Paclitaxel-Cyclodextrin-Dimer-Komplex.

Die folgenden Beispiele sollen das erfindungsgemäße Verfahren weiter erläutern.

### Beispiel 1

### Synthese eines β-[1,6(A-D)] überkappten Cyclodextrins und seine weitere Umwandlung zu β(6)-Diamidopropyl-diamino-cyclodextrin

3 g im Ofen bei 105°C getrocknetes β-Cyclodextrin (WACKER Chemie, Burghausen) wurden in 50 ml Pyridin gelöst. Eine Lösung von 1 g 4,4-Methylen-bis(benzolsulfonsäure)dichlorid in 50 ml Pyridin wurde langsam zugetropft. Der Ansatz wurde bei Raumtemperatur für 3 Stunden gerührt. Dann wurden 10 ml Wasser zugesetzt und alle Lösungsmittel am Rotationsverdampfer bei 70°C entfernt. Der verbleibende Sirup wurde 2 x mit Wasser behandelt und evaporiert. Dann wurde in 10 ml Wasser gelöst und in 500 ml Aceton präzipitiert. MALDI Massenspektroskopie zeigte ein Molekulargewicht von (M+Na⁺) = 1451 (1).

Substanz (1) kann weiter durch Reversed Phase Chromatographie gereinigt werden.

Die Substanz wurde weiterbehandelt mit dem 10fachen Überschuß an Diaminopropan, um β-[6(A-D)]-Diamidopropan-diamino-cyclodextrin zu erhalten (2). Hierzu wurde eine Lösung der Substanz (1) in heißem Wasser in eine wäßrige Lösung von Diaminopropan eingetropft und bei 70°C für 3 Stunden gerührt. Das durch Evaporierung konzentrierte Produkt wurde in Aceton-Methanol 20:1 gefällt und weiter durch Soxhlet-Extraktion über Aceton-Methanol (4:1) nachgereinigt.

Eine weitere Reinigung gelingt durch Ionenaustauscherchromatographie an SP-Sephadex mit einem Gradienten Wasser auf 2 M Triethylamin in Wasser.

Analog können α- und γ-Cyclodextrin mit dem Reagenz umgesetzt und in geschilderter Weise weiterverarbeitet werden.

### Beispiel 2

### β-(2) Monotosylcyclodextrin und dessen Umsetzung zu β-(2) Cyclodextrin-(3-thio-propionsäure) oder β-(2) Cyclodextrin-(3-thiobrenztraubensäure) oder β-(2) Cyclodextrin-(2-thioessigsäure)

2,5 g nach Ruebner et al. (1997) präpariertes und gereinigtes 2-Monotosyl-β-cyclodextrin wurde in 40 ml Dimethylformamid (DMF) gelöst. 1,7 g 3-Mercapto-propionsäure wurde in 60 ml DMF gelöst, und 0,3 g trockenes K₂CO₃ wurde zugesetzt. Diese Lösung wurde auf 70°C erhitzt und die erste Lösung tropfenweise zugesetzt. Nach dreistündigem Rühren wurde die Lösung abgekühlt, filtriert und am Rotationsverdampfer eingedampft. Der sirupöse Rest wurde in Aceton gefällt und das Präzipitat getrocknet. Weitere chromatographische Reinigung erfolgte an QAE-Sephadex unter Elution mit einem Gradienten von 0 auf 2 M Ameisensäure. MALDI: (M + K⁺) = 1263.

Entsprechend können β-(2S) Cyclodextrin-(3thiobrenztraubensäure) unter Argonatmosphäre und β-(2S)-Cyclodextrin (2-thioessigsäure) präpariert werden.

### Beispiel 3

### Bildung von Trimeren aus den Substanzen der Beispiele 1 und 2

Mehrere Methoden zur Präparation von Cyclodextrin-Trimeren wurden mit den Substanzen der Beispiele 1 und 2 erfolgreich versucht.
(A) Produkt aus Beispiel 2 wurde in einem geringen Volumen DMF gelöst, ein 20facher molarer Überschuß an Carbonyldiimidazol zugesetzt und die Lösung für 30 min auf 70°C erhitzt. Eine kleine Menge N-Hydroxysuccinimid wurde zugesetzt. Danach wurde der 2 1/2 fache molare Überschuß an Substanz aus Beispiel 1 zugesetzt und die Lösung für 2 Stunden gerührt. Das Produkt wurde im Vakuum konzentriert und mit Aceton gefällt. Weitere Reinigung durch sequentielle Ionenaustauscherchromatographie an SP- und QAESephadex lieferte das reine Trimer.
(B) Produkt aus Beispiel 2 wurde in einem kleinen Volumen wäßrigem Phosphatpuffer (pH 5,5) gelöst und ein 20facher molarer Überschuß an EDAC und eine geringe Menge N-Hydroxysuccinimid zugesetzt. Nach 30 min wurde ein 2,5facher molarer Überschuß an Substanz aus Beispiel 1 in wäßriger Lösung zugetropft. Der Reaktionsansatz wurde unter Rühren bei 4°C für 4 Tage im Kühlraum belassen. Weitere Reinigung wie unter (A).
(C) Gereinigte überkappte Cyclodextrine konnten direkt mit Cysteamin in DMF zu di-Cysteaminyl-cyclodextrinen umgesetzt werden. Die Produkte wurden in Aceton präzipitiert und weiter mit 2-Tosyl-β-cyclodextrin bei 70°C in DMF umgesetzt. Diese Kurzketten-Trimere konnten weiter mittels Reversed-Phase-Chromatographie an Lichroprep RP8 gereinigt werden.

### Beispiel 4

### Bildung physikalischer Einschlußkomplexe zwischen pharmazeutischem Wirkstoff und trimeren Cyclodextrinen entsprechend Beispiel 3. Generelle Methode und Reinigung der Komplexe

Die pharmazeutischen Wirkstoffe wurden zur Komplexierung in Ethanol oder Dimethylsulfoxid gelöst und zu einer Substanz entsprechend Beispiel 3 in Wasser oder phosphatgepufferter physiologischer Kochsalzlösung (PBS) zugesetzt. Die Lösungen wurden am Rotationsverdampfer auf 70°C erwärmt und zur Entfernung des Ethanols kurz evakuiert. Die Lösungen wurden nach Abkühlen auf eine Säule mit TSK-Gel gegeben und mit Wasser oder PBS eluiert. Die ersten (unter Umständen gefärbten) Fraktionen enthalten den reinen Komplex.

### Beispiel 5

### Durchführung biologischer Tests mit Komplexen entsprechend Beispiel 4, injektionen und Infusionen bei Versuchstieren

Gereinigte Komplexe entsprechend Beispiel 4 wurden in Mäuse oder Ratten in Dosen von 2 bis 5 mg/kg entsprechend der quantitativen Bestimmung mit Fluoreszenz- oder Absorptionsspektrometrie intravenös injiziert. Urin und Faeces der Tiere wurden gesammelt. Nach 24 Stunden wurden die Tiere getötet und ihre Organe zur Fluoreszenzanalyse aufgearbeitet. Dies geschieht vorzugsweise durch Extraktion mit Methanol:Aceton 1:1, da die erfindungsgemäßen Komplexe mit Hilfe dieser Lösungsmittel dissoziert werden können. Weniger effektiv ist die Auflösung von Organhomogenaten in 2% Natriumdodecylsulfat in wäßriger Lösung, gefolgt von Fluoreszenzspektroskopie. Hierdurch lassen sich die Menge des gebundenen Gastmoleküls in verschiedenen Organen bestimmen und so Schlußfolgerungen auf die Pharmakokinetik der Komplexe erarbeiten.

### Beispiel 6

### Verkapselung von Taxol in das CD-Dimer

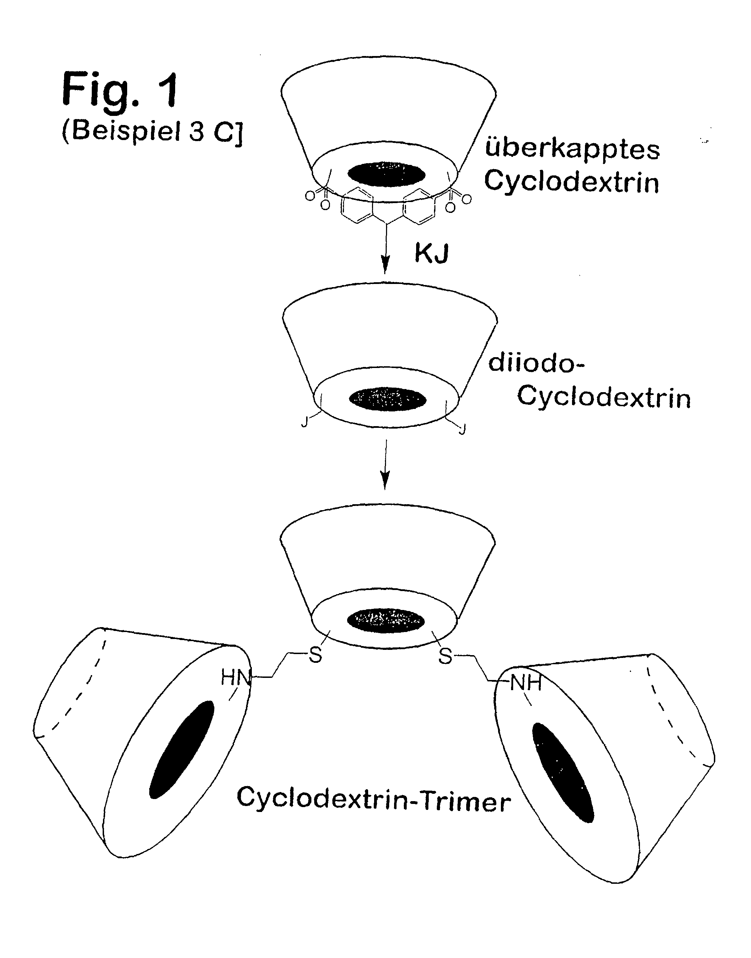

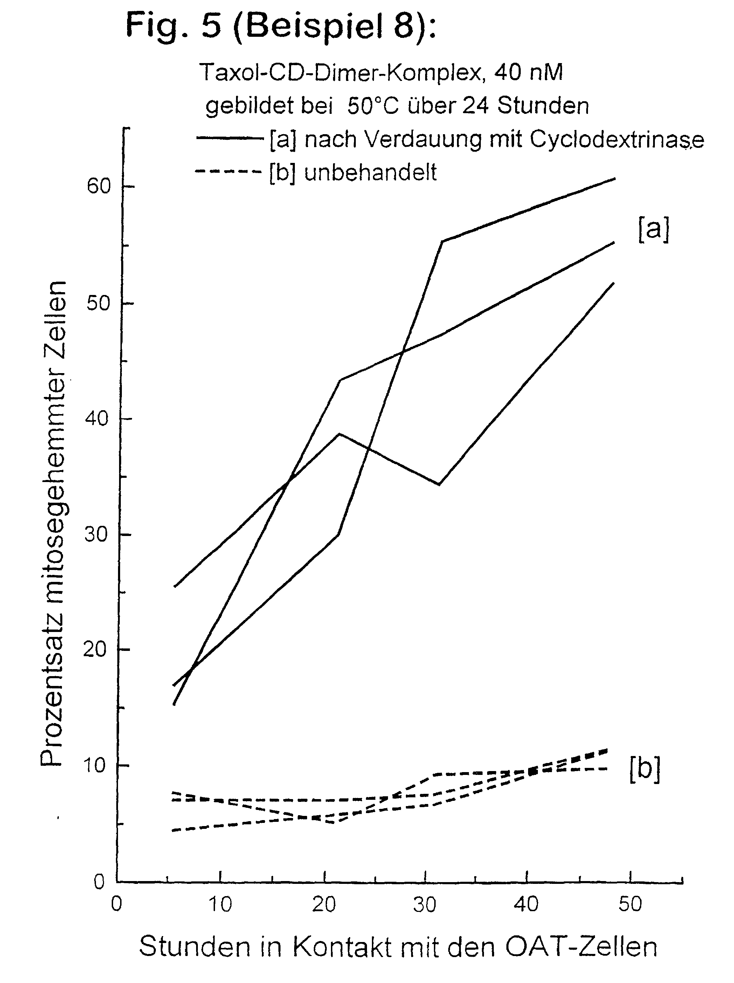

250 mg (100 µMol) des CD-Dimers Di-β-CD(2N-A4C5A4) (siehe Figur 5) wurden in 1 l wäßrigem Puffer (pH 7,35, Hepes 25 mM) von 60°C gelöst und unter starkem Rühren bei dieser Temperatur belassen. 8,5 mg Paclitaxel (10 µMol) wurden in 1 ml Dimethylsulfoxid gelöst und unter starkem Rühren tropfenweise dem Ansatz zugefügt. Der Ansatz blieb unter Rühren für 24 Stunden bei 60°C. Danach wurde abgekühlt und der Ansatz gefriergetrocknet. Das Fluoreszenspektrum (Figur 1) zeigt die Monomerisierung des komplexierten Taxols.

In Konkurrenzreaktion mit Toluidino-naphthalinsulfonsäure findet man eine Komplexbindungskonstante Kₜₐₓₒₗ = 1,4 µM. Die Komplexbildung kann in gleicher Weise mit CD-Dimeren, die über einen C5-Spacer (Cadaverin) Biotin tragen, durchgeführt werden. Die Bindungsfähigkeit des Biotins an Avidin leidet nicht durch die Behandlung bei 60°C.

### Beispiel 7

### Reinigung des Komplexes durch Gelchromatographie an TSK-Gel

Der Ansatz wurde in 10 ml Wasser gelöst und auf eine Chromatographiesäule 3 x 100 cm, gefüllt mit TSK-Gel, aufgetragen. Bei fraktionierter Elution mit Wasser enthält der 1. Peak den reinen, von Begleitstoffen gereinigten Komplex. Die Detektion erfolgt im Durchfluß bei 260 oder 280 nm. Das Produkt wurde aus dem Eluat durch Filtration über ein steriles Filter mit 0,2 µm Porenweite sterilisiert.

### Beispiel 8

### Prüfung des Komplexes auf Toxicität in der Zellkultur

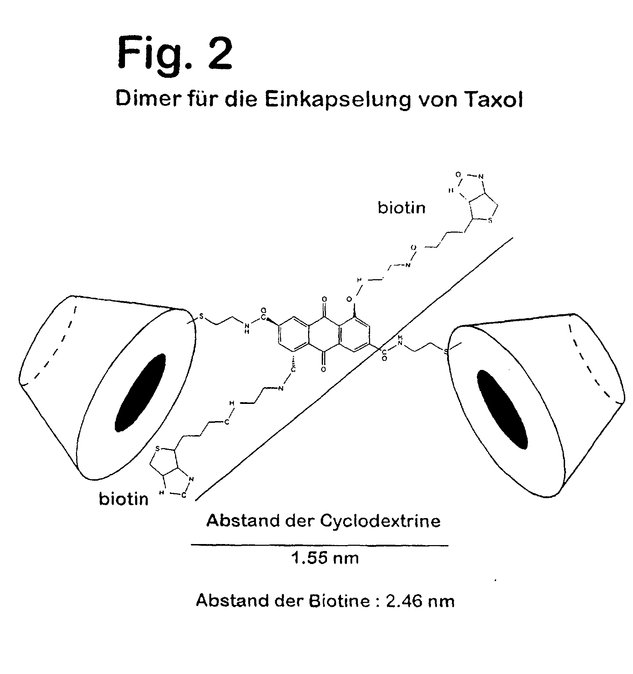

1/1000 des gemäß Beispiel 2 gereinigten Komplexes (= 10 nmol Paclitaxel) wurden in 250 ml DMEM-Kulturmedium gelöst (Endkonzentration 40 nM). Ein gleicher Ansatz wurde mit nicht-verkapseltem Taxol bereitet. Die Ansätze wurden zu Kulturzellen (OAT SCLC) gegeben und die Anzahl der abgerundeten Zellen (= Zellen in gehemmter Mitose) alle 3 Stunden ausgezählt. Das komplexierte Taxol zeigte keine mitosehemmende Wirkung innerhalb von 24 Stunden auf die Kulturzellen (Figur 2).

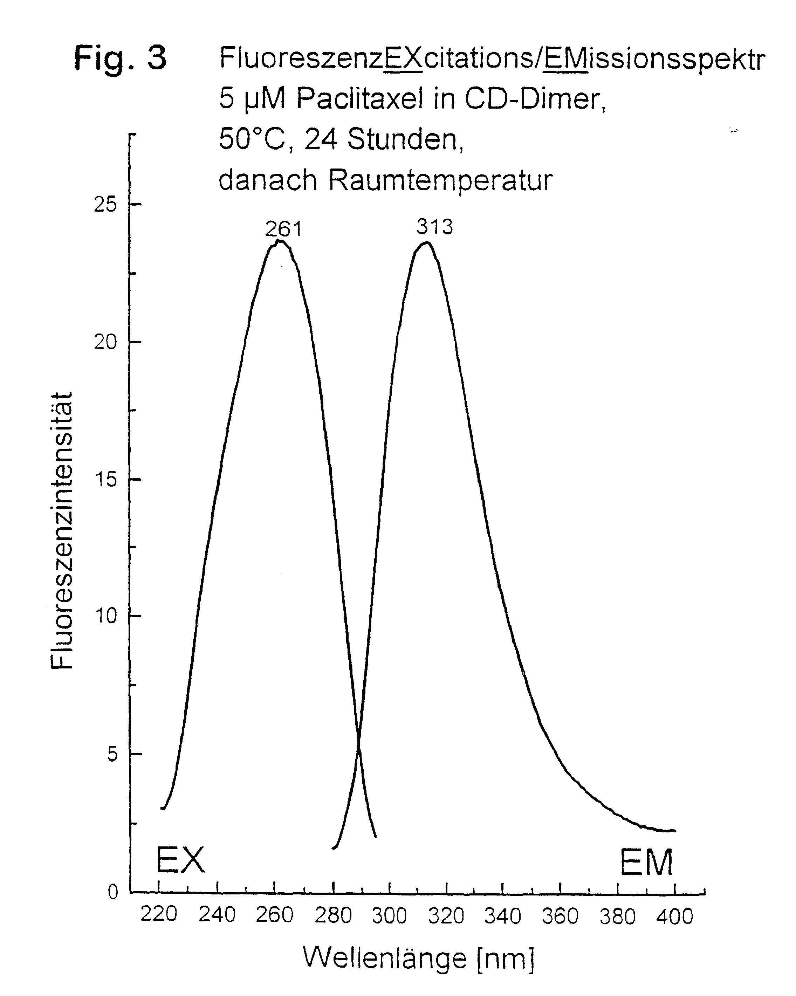

Die Behandlung des Komplexes mit dem Enzym Cyclodextrinase (aus Klebsiella oxytoca) zeigte bei gleicher Versuchsanordnung die Freisetzung und Reaktivierung des komplexierten Taxols aus dem Komplex (Figur 3).

### Beispiel 9

### Spezifisches Targeting des Taxol-Komplexes auf Tumoren der Maus

Xenotransplantierte OAT SCLC Zellen auf der Nacktmaus wurden für 1 Woche zur Tumorbildung belassen. Danach wurden die Mäuse für 24 Stunden mit dem biotinylierten monoklonalen Antikörper ICO 25 durch intraperitoneale Injektion von 1 mg Antikörper pro Maus vorbehandelt. Sodann wurden 5 mg NeutrAvidin intraperitoneal injiziert. Nach weiteren 48 Stunden wurde der aus biotinyliertem (BiotinCadaverin)-CD-Dimer und Paclitaxel gebildete Komplex (1-2-5 mg/Maus) injiziert und das Wachstum des Tumors über 4 Wochen verfolgt. Die behandelten Tumoren wachsen maximal mit 15% der Wachstumsgeschwindigkeit unbehandelter Tumoren (1-2 mg/Maus) oder werden ganz eliminiert (5 mg/Maus). Nebenwirkungen, die bei nicht-verkapseltem Taxol auftreten, werden nicht beobachtet.

## Patentansprüche

1. Cyclodextrin-Oligomer mit der allgemeinen Formel
CD-X-A-X-B-X-A-X-CD,
wobei
X jeweils unabhängig voneinander ausgewählt wird aus -NH-, -O-, -S-, -CO- oder kovalenter Bindung;
A jeweils ein aliphatischer C₂ bis C₄-Rest oder eine kovalente Bindung ist;
B ausgewählt wird aus Melamin, Trimesinsäure, Alizarintetracarbonsäure oder Tetraamino-Alizarin, α-Cyclodextrin, β-Cyclodextrin, γ-Cyclodextrin, gebunden über die Primärseite, sowie
CD jeweils ein über die Sekundärseite gebundenes Cyclodextrin darstellt.

2. Cyclodextrin-Oligomer nach Anspruch 1, **dadurch gekennzeichnet, daß** die Substanz
Di-β-CD(-NHCO(Trimesyl)CONH-),
Di-β-CD(-NH-(C₂H₄)-NHCO(Trimesyl)CONH-(C₂H₄)-NH-),
Di-β-CD(-NH-(C₂H₄)-SH-[6-α-CD-6]-SH-(C₂H₄)-NH-),
Di-β-CD(-NH-(C₂H₄)-SH-[6-(β,γ)-CD-6]-SH-(C₂H₄)-NH-) oder
Di-β-CD(-NH-(C₃H₆)-NHCO(Trimesyl)CONH-(C₃H₆)-NH-) ist.

3. Cyclodextrin-Oligomer nach einem der Ansprüche 1 bis 2, **dadurch gekennzeichnet, daß** das Cyclodextrin-Oligomer zusätzlich mindestens eine Affinitätsgruppierung trägt, die mit molekularen Zielstrukturen in Wechselwirkung treten können.

4. Cyclodextrin-Oligomer nach einem der Ansprüche 1 bis 3, **dadurch gekennzeichnet, daß** das Cyclodextrin-Oligomer zusätzlich zwei Affinitätsgruppierung trägt, die mit molekularen Zielstrukturen in Wechselwirkung treten können.

5. Cyclodextrin-Oligomer nach Anspruch 3 oder 4, **dadurch gekennzeichnet, daß** die mindestens eine Affinitätsgruppierung an die Einheit B des Spacers kovalent gebunden ist.

6. Cyclodextrin-Oligomer nach einem der Ansprüche 3 bis 5, **dadurch gekennzeichnet, daß** die mindestens eine Affinitätsgruppierung ein Biotinylrest oder ein Digoxin-/Digoxigeninrest ist.

7. Komplex aus einem Cyclodextrin-Oligomer gemäß einem der Ansprüche 1 bis 6 und einem pharmazeutischen Wirkstoff.

8. Komplex gemäß Anspruch 7, **dadurch gekennzeichnet, daß** der pharmazeutische Wirkstoff ein hohes Nebenwirkungspotential hat.

9. Komplex gemäß Anspruch 7 oder 8, **dadurch gekennzeichnet, daß** der pharmazeutische Wirkstoff Mitosehemmer, Tumortherapeutika oder Photochemotherapeutika sind.

10. Komplex gemäß einem der Ansprüche 7 bis 9, **dadurch gekennzeichnet, daß** der pharmazeutische Wirkstoff Taxol, ein Taxolderivat, Colchizin, Colchemid, 3¹,8¹-(di-t-butyl-phenoxy)porphyrine, Phenytoin, Chlortrianisen, Tamoxifen, Vinblastin, Vincristin oder Docetaxel ist.

11. Verfahren zur Herstellung der Komplexe gemäß einem der Ansprüche 7 bis 10, wobei ein Cyclodextrin-Oligomer gemäß einem der Ansprüche 1 bis 6 mit einem einzuschließenden pharmazeutischen Wirkstoff bei erhöhter Temperatur in Kontakt gebracht wird.

12. Verfahren zur Herstellung der Cyclodextrin-Oligomere gemäß einem der Ansprüche 1 bis 6, wobei Cyclodextrine auf der Sekundärseite mittels Aktivierung der OH-Gruppen und Umsetzung mit kurzen bifunktionellen Spacergruppen funktionalisiert werden und dann mit den bifunktionellen Spacern umgesetzt werden.

13. Arzneimittel enthaltend Cyclodextrin-Oligomere gemäß einem der Ansprüche 1 bis 6 oder einen Komplex gemäß den Ansprüchen 7 bis 10.

14. Verwendung Arzneimittels zur Behandlung von der Cyclodextrin-Oligomere gemäß einem der Ansprüche 1 bis 6 oder der komplexe gemäß einem der Ansprüche 7 bis 10 zur Herstellung eines Tumorerkrankungen.

15. Verwendung nach Anspruch 14, wobei die Tumorerkrankung ausgewählt ist aus der Gruppe Blasentumore, Mammacarcinome, Uteruscarcinome, Oesophaguscarcinome, Magencardnome, Kopf-Hals-Carcinome, Nasopharynxcarcinom, Hepatocell. Carcinome, Lungencarcinome, Lymphome, Melanome, Ovarialcarcinome und Prostatacarcinome.

## Claims

1. A cyclodextrin oligomer having the general formula
CD-X-A-X-B-X-A-X-CD
where
each X is independently selected from -NH-, -O-, -S-, -CO- or a covalent bond;
each A is an aliphatic C₂ to C₄ residue or a covalent bond;
B is selected from melamine, trimesic acid, alizarintetracarboxylic acid or tetraaminoalizarin, α-cyclodextrin, β-cyclodextrin or γ-cyclodextrin bound through its primary side; and
each CD represents a cyclodextrin bound through its secondary side.

2. The cyclodextrin oligomer according to claim 1, **characterized in that** the substance is
di-β-CD(-NHCO(trimesyl)CONH-);
di-β-CD(-NH-(C₂H₄)-NHCO(trimesyl)CONH-(C₂H₄)-NH-);
di-β-CD(-NH-(C₂H₄)-SH-[6-α-CD-6]-SH-(C₂H₄)-NH-);
di-β-CD(-NH-(C₂H₄)-SH-[6-(β,γ)-CD-6]-SH-(C₂H₄)-NH-); or
di-β-CD(-NH-(C₃H₆)-NHCO(trimesyl)CONH-(C₃H₆)-NH-).

3. The cyclodextrin oligomer according to any of claims 1 to 2, **characterized in that** said cyclodextrin oligomer additionally carries at least one affinity group which can interact with molecular target structures.

4. The cyclodextrin oligomer according to any one of claims 1 to 3, **characterized in that** said cyclodextrin oligomer carries two additional affinity groups which can interact with molecular target structure.

5. The cyclodextrin oligomer according to claim 3 or 4, **characterized in that** said at least one affinity group is covalently bound to the unit B of the spacer.

6. The cyclodextrin oligomer according to any one of claims 3 to 5, **characterized in that** said at least one affinity group is a biotinyl residue or a digoxin/digoxigenin residue.

7. A complex of a cyclodextrin oligomer according to any of claims 1 to 6 with a pharmaceutically active substance.

8. The complex according to claim 7, **characterized in that** said pharmaceutically active substance has a high potential for side-effects.

9. The complex according to claim 7 or 8, **characterized in that** said pharmaceutically active substance are mitotic inhibitors, tumor therapeutics or photochemotherapeutics.

10. The complex according to any one of claims 7 to 9, **characterized in that** said pharmaceutical active substance is taxol, a taxol derivative, colchicine, colchemide, 3¹,8¹-(di-t-butylphenoxy)porphyrin, phenytoin, chlorotrianisene, tamoxifene, vinblastin, vincristin or docetaxel.

11. A method for the preparation of the complexes according to any of claims 7 to 10 by contacting a cyclodextrin oligomer according to any of claims 1 to 6 with a pharmaceutically active substance to be enclosed at an elevated temperature.

12. A method for the preparation of the cyclodextrin oligomers according to any of claims 1 to 6 by functionalizing cyclodextrins on their secondary sides by activation of the OH groups and reaction with short bifunctional spacer groups, followed by reaction with said bifunctional spacers.

13. A medicament containing cyclodextrin oligomers according to any of claims 1 to 6 or a complex according to claims 7 to 10.

14. Use of said cyclodextrin oligomer according to any one of claims 1 to 6 or a complex according to any one of claims 7 to 10 for the preparation of a medicament for the treatment of tumor diseases.

15. Use of the medicament according to claim 14 for the treatment of tumor diseases, such as tumors of the bladder, carcinomas of the breast or uterus, esophageal cancers, gastric carcinomas, cephalo-cervical carcinomas, naso-pharynx carcinoma, hepatocellular carcinomas, pulmonary carcinomas, lymphomas, melanomas, ovarial carcinomas and prostatic carcinomas.

## Revendications

1. Oligomère de cyclodextrine ayant la formule générale
CD-X-A-X-B-X-A-X-CD,
dans laquelle
X est choisi, à chaque fois indépendamment les uns des autres, parmi -NH-, -O-, -S-, -CO- ou une liaison covalente;
A est à chaque fois un résidu aliphatique en C₂ à C₄ ou une liaison covalente ;
B est choisi parmi la mélamine, l'acide trimésique, l'acide alizarinetétracarboxylique ou une tétraamino-alizarine, une α-cyclodextrine, une β-cyclodextrine, une γ-cyclodextrine, lié au moyen du côté primaire, et
CD représente à chaque fois une cyclodextrine liée au moyen du côté secondaire.

2. Oligomère de cyclodextrine selon la revendication 1, **caractérisé en ce que** la substance est
Di-β-CD(-NHCO-trimésyl)CONH-),
Di-β-CD(-NH-(C₂H₄)-NHCO(trimésyl)CONH-(C₂H₄)-NH-),
Di-β-CD(-NH-(C₂H₄)-SH-[6-α-CD-6]-SH-(C₂H₄)-NH-),
Di-β-CD(-NH-(C₂H₄)-SH-[6-(β,γ)-CD-6]-SH-(C₂H₄)-NH-) ou
Di-β-CD(-NH-(C₃H₆)-NHCO(trimésyl)CONH-(C₃H₆)-NH-).

3. Oligomère de cyclodextrine selon l'une des revendications 1 et 2, **caractérisé en ce que** ledit oligomère de cyclodextrine porte, en outre, au moins un groupement d'affinité qui peut entrer en interaction avec des structures moléculaires ciblées.

4. Oligomère de cyclodextrine selon l'une des revendications 1 à 3, **caractérisé en ce que** ledit oligomère de cyclodextrine porte, en outre, deux groupements d'affinité qui peuvent entrer en interaction avec des structures moléculaires ciblées.

5. Oligomère de cyclodextrine selon la revendication 3 ou 4, **caractérisé en ce que** le groupement d'affinité, au nombre d'au moins un, est lié à l'unité B du segment espaceur par covalence.

6. Oligomère de cyclodextrine selon l'une des revendications 3 à 5, **caractérisé en ce que** le groupement d'affinité, au nombre d'au moins un, est un résidu biotinyle ou un résidu digoxine/digoxigénine.

7. Complexe formé d'un oligomère de cyclodextrine selon l'une des revendications 1 à 6 et d'un principe actif pharmaceutique.

8. Complexe selon la revendication 7, **caractérisé en ce que** le principe actif pharmaceutique a un grand potentiel d'effet secondaire.

9. Complexe selon la revendication 7 ou 8, **caractérisé en ce que** le principe actif pharmaceutique est constitué d'inhibiteurs de mitose, d'agents thérapeutiques antitumoraux ou d'agents thérapeutiques photochimiques.

10. Complexe selon l'une des revendications 7 à 9, **caractérisé en ce que** le principe actif pharmaceutique est du taxol, un dérivé du taxol, de la colchicine, du colchicamide, de la 3¹,8¹-(di-t-butyl-phénoxy)porphyrine, de la phénytoïne, du chlortrianisène, du tamoxifène, de la vinblastine, de la vincristine ou du docétaxel.

11. Procédé de préparation des complexes selon l'une des revendications 7 à 10, dans lequel un oligomère de cyclodextrine selon l'une des revendications 1 à 6 est amené au contact d'un principe actif pharmaceutique à inclure à une température élevée.

12. Procédé de préparation des oligomères de cyclodextrine selon l'une des revendications 1 à 6, dans lequel des cyclodextrines sont fonctionnalisées du côté secondaire par activation des groupes OH et par mise en réaction avec des groupes espaceurs bifonctionnels courts et, ensuite, sont mises en réaction avec les segments espaceurs bifonctionnels.

13. Médicament contenant des oligomères de cyclodextrine selon l'une des revendications 1 à 6 ou un complexe selon les revendications 7 à 10.

14. Utilisation des oligomères de cyclodextrine selon l'une des revendications 1 à 6 ou des complexes selon l'une des revendications 7 à 10 pour la préparation d'un médicament pour le traitement de maladies tumorales.

15. Utilisation selon la revendication 14, dans laquelle la maladie tumorale est choisie dans l'ensemble constitué des tumeurs de la vessie, du cancer du sein, du cancer de l'utérus, du cancer de l'oesophage, du cancer de l'estomac, du cancer du cerveau et de la gorge, du cancer du naso-pharynx, des hépatomes, du cancer du poumon, des lymphomes, des mélanomes, du cancer de l'ovaire et du cancer de la prostate.
